(19) Europäisches Patentamt
European Patent Office
Office européen des brevets

(11) Veröffentlichungsnummer: 0 400 686
A1

# (12) EUROPÄISCHE PATENTANMELDUNG

(21) Anmeldenummer: 90113147.4

(51) Int. Cl.[5]: C07D 473/32, A61K 31/52

(22) Anmeldetag: 13.09.86

(30) Priorität: 24.09.85 DE 3533983
02.05.86 DE 3614902
09.08.86 DE 3627024

(43) Veröffentlichungstag der Anmeldung:
05.12.90 Patentblatt 90/49

(60) Veröffentlichungsnummer der früheren Anmeldung nach Art. 76 EPÜ: 0 217 207

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Winkelmann,Erhardt,Dr.
Brunhildenweg 5
D-6233 Kelkheim(Taunus)(DE)
Erfinder: Rolly,Heinrich,Dr.
Am Tulpengarten 18
D-6233 Kelkheim(Taunus)(DE)
Erfinder: Winkler,Irvin,Dr.
In den Eichen 40
D-6237 Liederbach(DE)

## (54) 2-Amino-9-[1,3-bis-(isopropoxy)-propyl-2-oxymethyl]-purin, seine Verwendung und diese Verbindung enthaltende Arzneimittel.

(57) 2-Amino-9-[1,3-bis(isopropoxy)-propyl-2-oxymethyl]-purin eignet sich zur Bekämpfung von Erkrankungen, die durch Viren verursacht werden.

EP 0 400 686 A1

Xerox Copy Centre

## 2-Amino-9-[1,3-bis-(isopropoxy)-propyl-2-oxymethyl]-purin, seine Verwendung und diese Verbindung enthaltende Arzneimittel

Es sind Arzneimittel bekannt, die 2-Amino-6-hydroxy-9-(2-hydroxyäthoxymethyl)purin (Acyclovir - vgl. Figur 1, Formel ACV in der tautomeren Oxoschreibweise) als Wirkstoff enthalten. Diese stellen gute Antiherpes-Mittel dar (vgl. EP-OS 72 027).

Später sind Arzneimittel gegen virale Infektionen entwickelt worden, die sich von denen der Formel ACV dadurch unterscheiden, daß sie in Nachbarstellung zu der Äthergruppe im Hydroxyäthylrest noch zusätzlich eine Hydroxymethylgruppe enthalten, die also ein Glycerinderivat darstellen, wobei der Sauerstoff in 6-Stellung auch zusätzlich durch Schwefel ersetzt sein kann. (vgl. J. Med. Chem. 28,358 (1985)). Derartige Verbindungen sind schließlich weiter dahingehend modifiziert worden, daß die Hydroxymethylgruppe noch durch eine Benzylgruppe veräthert worden ist und gegebenenfalls der Sauerstoff in 6-Stellung durch eine Iminogruppe ersetzt ist (vgl. EP-OS 152 965).

Gegenstand der Erfindung ist nun das 2-Amino-9-[1,3-bis-(isopropoxy)-propyl-2-oxymethyl]-purin Verbindungen der Formel 1

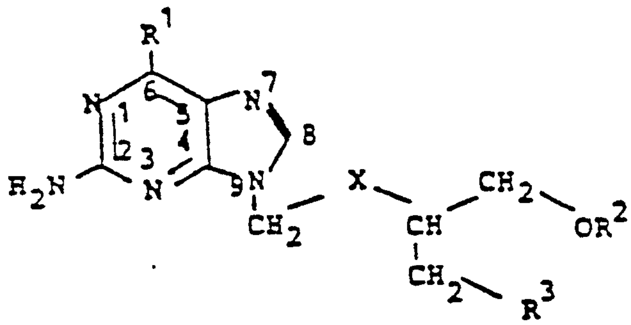

1

in denen $R^1$ OH oder SH, $R^2$ H, $R^3$ Y-$R^4$, X O oder S, Y O und $R^4$ Alkyl mit 1 bis 8 C-Atomen darstellen, sind aus der EP-OS 145 207 bekannt und auch ihre Verwendung als Arzneimittel für die Behandlung von autoimmunen Krankheiten wie Arthritis, multipler Sklerose usw. sowie von viralen Infektionen und Krebs. Neben diesen Verbindungen werden in der genannten EP-OS 145 207 auch noch, und zwar hauptsächlich, solche beschrieben und beansprucht, die in 8-Stellung ein Bromatom oder eine $NH_2$- bzw. $NHCOR^6$-Gruppe enthalten, wobei $R^6$ einen $C_1$-$C_4$-Alkylrest, einen Arylrest oder einen Aralkylrest darstellen kann. Den Verbindungen, die in 8-Stellung Wasserstoff enthalten, werden zwar ebenfalls die obengenannten Wirkungen zugeschrieben, jedoch werden sie vor allem als Zwischenprodukte zur Herstellung der in 8-Stellung substituierten Verbindungen beschrieben und benötigt (vgl. Schema 1, Reaktionsschritte 22(2),23-(2), 24(3), 25(4) und 11 (5) sowie Schema 2, Reaktionsschritte 34(2), 33(2), 36(3), 37(4) und 26(5)). Pharmakologische Daten bezüglich der Wirkungsweise werden in der EP-OS 145 207 weder für die in 8-Stellung substituierten Verbindungen noch für die in 8-Stellung unsubstituierten Verbindungen angegeben.

Nach dem Schema 1 der EP-OS 145 207, Reaktionsschritt 22(2) zu 23(2), wird der Benzylrest als Schutzgruppe durch katalytische Hydrierung abgespalten. Derartige Reaktionen sind mit einer Alkylgruppe, wie Isopropylgruppe nicht möglich, weil unter den dort beschriebenen Reaktionsbedingungen zwar die Benzylgruppe als Toluol abgespalten werden kann, nicht aber aliphatische Äthergruppen.

Gegenüber den aus der EP-OS 145 207 bekannten, in 8-Stellung substituierten und in 8-Stellung unsubstituierten Verbindungen und auch gegenüber der Verbindung der Formel Z haben die erfindungsgemäßen Verbindungen der Vorteil der erheblich größeren Wirksamkeit. Diese bedeutet, daß bei gleicher bzw. verbesserter Toxizität wesentlich geringere Mengen der Wirkstoffe verwendet werden können.

Die erfindungsgemäßen Verbindungen der allgemeinen Formel 1 können nach verschiedenen Methoden hergestellt werden, wobei man je nach Bedeutung des Restes $R^1$ verschiedene Ausgangsstoffe und geeignete Schutzgruppen wählt. Einige Ausfüh rungsformen (A bis D) und die im folgenden angegebenen Formelbezeichnungen sind im Reaktionsschema (Figur 2) wiedergegeben, worin HMDS Hexamethyldisilazan, Pyr Pyridin und Ac Acetyl bedeutet und bei den Formeln, in denen $R^1$ OH, SH oder $NH_2$ bedeutet, auch eine Schreibweise in tautomerer Form gemäß Formeln 2′, 3′ und 6′ (s. Figur 1) möglich wäre. In den Fällen, in denen mit Halogenverbindungen umgesetzt wird, ist das Halogen bevorzugt Chlor, wenngleich es auch ein anderes, wie Brom, sein kann.

Die als Ausgangsstoff verwendeten Purine sind bekannt und überwiegend käuflich, so z.B.: 2-Amino-6-hydroxy-purin = Guanin, 2-Amino-6-mercapto-purin und 2-Amino-6-chlor-purin (beide in J.Med.Chem. 28 - (3), 358 (1985)), 2,6-Diamino-purin (Synthesis 11, 963 (1984), EP-OS 138 683) und 2-Amino-purin (EP-OS 108 285). Die Ausgangsstoffe der Formeln 13 und 14 sind überwiegend bekannt und nach bekannten Methoden herstellbar (siehe Beispiele 1a-d und 3a-g).

Verfahren A)

Wenn $R^1$ Hydroxy (bzw. in der tautomeren Form Oxo) (Formel 2), Amino (bzw. Imino) (Formel 6), Wasserstoff (Formel 7) oder $R^1$ = Z-$R^5$ (Formel 21) - s. jeweils Figur 2- ist, setzt man zweckmäßig 2,6,9-Tris-(trimethylsilyl)- bzw. 2,9-Bis-(trimethylsilyl)-2-amino-purine gemäß einer der Formeln 8, 11 und 12 mit Halogen-, insbesondere Chlorverbindungen der Formel 13 um, worin $R^2$ = Acyl ist und X und $R^3$ die angegebene Bedeutung haben, spaltet die Trimethylsilyl-Schutzgruppen ab und erhält so in 9-Position substituierte Verbindungen der Formel 1, speziell der Formeln 15, 19, 20 und 22. Dabei zeichnet sich in Formel 13 das in α-Stellung zu X stehende Halogen, insbesondere Chlor durch eine besondere Reaktivität aus. Natürlich kann man anstelle der Trimethylsilylschutzgruppe auch andere Trialkylsilylschutzgruppen verwenden, in denen der Alkylrest z.B. 1 bis 6, vorzugsweise 1 bis 3 C-Atome hat und geradkettig oder verzweigt ist.

Die erfindungsgemäßen Verbindungen 15, 19 und 20 werden gegenbenenfalls zu anderen Verbindungen der Formel 1, in denen $R^2$ statt Acyl Wasserstoff ist, verseift (s. Figur 2).

Verfahren B)

Wenn $R^1$ Hydroxy (bzw. Oxo) (Formel 9), Mercapto (bzw. Thio) (Formel 10), Amino (bzw. Imino) (Formel 6a) oder Wasserstoff (Formel 7a) - s. jeweils Figur 1 ist, setzt man entsprechende Di- bzw. Triacyl-Verbindungen der Formeln 9, 10, 6a bzw. 7a mit reaktiven Acetoxy-Verbindungen der Formel 14 um, worin $R^2$ Acyl ist und X und $R^3$ die angegebene Bedeutung haben. Man erhält so in 9-Position substituierte Verbindungen der Formeln 16, 17, 6b und 7b. Diese erfindungsgemäßen Verbindungen werden gegebenenfalls zu anderen Verbindungen der Formel 1 verseift, in denen $R^2$ Wasserstoff ist.

Verfahren C)

Wenn $R^1$ Halogen, insbesondere Chlor ist (Formel 5), kann man $C_1$) diese Verbindung mit aktiven Halogenverbindungen der Formel 13 zu in 9-Position substituierten Verbindungen der Formel 18 umsetzen und diese $C_2$) gegebenenfalls zu anderen Verbindungen der Formel 1, in denen $R^2$ H ist, verseifen, oder $C_3$) vor oder nach der Verseifung zur Herstellung anderer Verbindungen der Formel 1 das Halogen austauschen, z.B. wenn
$R^1$ Hydroxy (bzw. Oxo) sein soll, durch saure Verseifung, z.B. mit wäßriger Salzsäure,
$R^1$ Mercapto (bzw. Thio) sein soll, durch Umsetzung mit Schwefelwasserstoff,
$R^1$ Azido sein soll, durch Umsetzung mit Alkaliaziden,
$R^1$ Amino (bzw. Imino) sein soll, durch Umsetzung mit Ammoniak,
$R^1$ Wasserstoff sein soll, durch katalytische dehalogenierende Hydrierung, z.B. mit Palladium-Kohle/Wasserstoff + Triäthylamin,
$R^1$ $ZR^5$ sein soll, durch Umsetzung mit Alkoholen bzw. Phenolen, Mercaptanen bzw. Thiophenolen oder Aminen bzw. Anilinen,
wobei $R^2$ Acyl oder gegebenenfalls nach Verseifung Wasserstoff ist und $R^3$ und X die angegebene Bedeutung haben.

Bevorzugt für das Verfahren C sind vielfach Verbindungen, in denen $R^1$ H, OH oder $NH_2$, $R^2$ Acyl, insbesondere Acetyl, und X O oder S sind, wobei $R^3$ und damit auch Y und $R^4$ die im Anspruch 1 angegebene Bedeutung haben.

Die Umsetzungen nach den Verfahren A bis C und dem weiter unten genannten Verfahren D, die Analogieverfahren darstellen, werden unter den üblichen Bedingungen durchgeführt. Bei den Verfarhren A) bis C) liegen die Reaktionstemperaturen im allgemeinen zwischen 0 und 150 °C, vorzugsweise zwischen 50 und 100 °C. Gewöhnlich arbeitet man bei Normaldruck, jedoch ist es auch möglich, bei erhöhtem oder vermindertem Druck zu arbeiten, wenngleich hiermit normalerweise keine Vorteile verbunden sind. Die Lösungs- und Verteilungsmittel können natürlich sowohl in Form einheitlicher Stoffe als auch in Form von

Gemischen verwendet werden. Zu den einzelnen Verfahren wird noch folgendes ausgeführt:

Die Umsetzungen nach Verfahren A) werden zweckmäßig in einem Lösungs- oder Verteilungsmittel durchgeführt. Dafür kommen insbesondere aprotische Lösungsmittel in Frage, wie Benzol, Toluol, die verschiedenen Xylole, ferner 1,2-Dichloräthan, Chlorbenzol, 1,2-Dimethoxyäthan, Dioxan. Zum Abfangen der gebildeten Halogenwasserstoffsäure empfiehlt sich die Verwendung einer Base, wie Triäthylamin oder N-Äthylmorpholin. Die Reaktionszeiten betragen je nach Reaktionstemperatur und Reaktionspartner einige Minuten bis mehrere Stunden, meistens 12-24 Stunden.

Wegen der Feuchtigkeitsempfindlichkeit der als Ausgangsstoffe verwendeten trialkylsilyl-geschützten Purine 8, 11, 12 empfiehlt sich die Verwendung eines Schutzgases im Reaktionsgefäß, z.B. Stickstoff oder Argon. Die Abspaltung der Trialkylsilyl-Schutzgruppen kann z.B. durch Kochen der Zwischenverbindung mit einem Alkohol, wie Methanol oder Äthanol erfolgen.

Die Umsetzungen nach Verfahren B) werden zweckmäßig ebenfalls in einen Lösungs- oder Verteilungsmittel durchgeführt. Dafür kommen insbesondere Sulfone als aprotische Lösungsmittel in Frage, wie Sulfolan (Tetramethylensulfon) oder Dipropylsulfon. Bei dieser Umsetzung empfiehlt sich die Verwendung eines sauren Katalysators, wie Methan-, Äthan-, Benzol- oder Toluol-sulfonsäure. Die Reaktionszeiten betragen je nach Reaktionstemperatur und Reaktionspartner meist mehrere Stunden bis zu einigen Tagen.

Die Umsetzungen nach Verfahren $C_1$ werden ebenfalls zweckmäßig in einem Lösungs- oder Verteilungsmittel durchgeführt. Dafür kommen insbesondere polare, aprotische Lösungsmittel in Frage, wie Dimethylformamid, Dimethylacetamid, Tetramethylharnstoff, Hexamethylphosphorsäuretriamid oder Dimethylsulfoxid. Zum Abfangen der gebildeten Halogenwasserstoffsäure empfiehlt sich die Verwendung einer Base, z.B. der weiter unten bei der Verseifung genannten, wie Triäthylamin, N-Äthylmorpholin oder Alkalicarbonate, wie Natrium- oder Kaliumcarbonat. Die Reaktionszeiten betragen je nach Reaktionstemperatur und Reaktionspartner mehrere Minuten bis mehrere Stunden.

Der Halogenaustausch nach Verfahren $C_2$ wird z.B. auf folgende Weise durchgeführt:

$C_2$a) Wenn $R^1$ Hydroxy (bzw. Oxo) ist, kann die Verseifung der Verbindung 18, z.B. mit 1-molarer wäßriger Salzsäure, durch 2-3-stündiges Kochen unter Rückfluß zur Verbindung 15 erfolgen; derartige Reaktionsbedingungen sind, ebenso wie die für das Verfahren $C_1$), eingehend in der Internationalen Anmeldung PCT/SE83/00254 beschrieben.

$C_2$b) Wenn $R^1$ Mercapto (bzw. Thio) ist, kann die Umsetzung der Verbindung 18 mit Schwefelwasserstoffgas in Gegenwart einer Base, wie Triäthylamin oder Pyridin, die gleichzeitig als Lösungsmittel dienen kann, oder in einem Alkohol wie Methanol, Äthanol, einem der Propanole oder Butanole, Äthylenglykolmonomethyläther oder -äthyläther, z.B. bei 20-60 °C, zur Verbindung 17, erfolgen.

$C_2$c) Wenn $R^1$ Azido ist, kann die Umsetzung mit Alkaliaziden in polaren, eventuell auch in unpolaren, gegenüber den Reaktionsteilnehmern inerten Lösungsmitteln wie Dimethylform- bzw. -acetamid, Dimethylsulfoxyd, Hexamethylphosphorsäuretriamid, N-Methylpyrrolidon, Acetonitril und Tetramethylharnstoff unter üblichen Bedinungen, z. B. zwischen 20 und 120, vorzugsweise zwischen 60 und 90 °C erfolgen.

$C_2$d) Wenn $R^1$ Amino (bzw. Imino) ist, kann die Umsetzung der Verbindung 18 z.B. mit Ammoniak (flüssig oder in alkoholischer Lösung), gegebenenfalls unter Verwendung eines gegenüber den Reaktionsteilnehmern inerten Lösungsmittels, wie Äthanol, im allgemeinen bei Temperaturen zwischen 20 und 120 °C, vorzugsweise 50-100 °C erfolgen, z.B. innerhalb von 12 Stunden.

$C_2$e) Wenn $R^1$ Wasserstoff ist, kann die dehalogenierende Hydrierung der Verbindung 18 vorteilhaft mit Palladium-Kohle/Wasserstoff unter atmosphärischem Druck in Gegenwart einer hierfür geeigneten Base, wie Triäthylamin, unter üblichen Bedingungen, z.B. in 12 Stunden bei Raumtemperatur, zur Verbindung 20 durchgeführt werden.

$C_2$f) Wenn $R^1$ Z-$R^5$, Z Sauerstoff, Schwefel oder NH und $R^5$ die angegebene Bedeutung haben, kann die Umsetzung der Verbindung 18 mit entsprechenden Hydroxy-, Mercapto- oder Aminoverbindungen erfolgen. Die Hydroxy- oder Mercaptoverbindungen werden in Form ihrer Alkalisalze, die Aminoverbindungen in Gegenwart einer anorganischen oder vorzugweise organischen Base, wie Triäthylamin oder Pyridin, zur Umsetzung gebracht. Als Lösungsmittel kann bei den Hydroxyverbindungen eingesetzter Alkohol im Überschuß, bei Aminoverbindungen z.B. die Hilfsbase Pyridin verwendet werden. Im allgemeinen verwendet man als Lösungsmittel Alkohole, wie Propanole und Butanole, Alkylenglykole oder ihre Mono- bzw. Dialkyläther. Die Temperaturen liegen im allgemeinen zwischen 25 und 130 °C, vorzugsweise zwischen 80 und 100 °C. Die Reaktionszeiten liegen je nach Temperatur und Reaktionsteilnehmer meistens zwischen 6 und 24 Stunden. Durch analoge Umsetzung der Verbindung 3 bzw. 5 mit ensprechenden Hydroxy-, Mercapto- oder Aminoverbindungen und weitere Umsetzung mit Verbindung 13 bzw. 1 nach Verfahren A) lassen sich Verbindungen mit $R^1$ = Z-$R^5$ (Verbindung 21 bzw. 22) herstellen, wie in Figur 2 dargestellt.

Verfahren D)

Soweit nach einem der Verfahren A bis C erfindungsgemäße Verbindungen wie 15 bis 20, 6b und 7b erhalten werden, können diese, z.B. durch Einwirkung von Basen, wie Ammoniak, Alkylaminen, wie Methyl- oder Äthylamin, den verschiedenen Propyl- oder Butylaminen, oder Alkalihydroxyden oder -carbonaten, wie Natriumhydroxid, Kaliumhydroxid, Natriumcarbonat, Kaliumcarbonat in wäßriger oder alkoholischer (Methanol, Äthanol) Lösung, im allgemeinen bei Temperaturen von 0-100 °C, vorzugsweise bei 20-50 °C verseift werden.

Die meisten Verbindungen der Formeln 15 bis 19 fallen nach Verfahren A bis D als Gemisch von Purin-7- und -9-Isomeren in wechselnden Mengenverhältnissen an, die gegebenenfalls durch fraktionierte Kristallisation getrennt werden können. Im allgemeinen wird die Isomerentrennung mittels Säulenchromatographie z.B. über Kieselgel durchgeführt. Die Charakterisierung der Isomeren und ihre Prozentgehaltbestimmung erfolgt z.B. durch Hochdruckflüssigkeitschromatographie (HPLC) unter Verwendung von (R)Li-Chrosorb Si 60/5 µm der Firma Merck, Darmstadt, Deutschland.

Die erfindungsgemäßen Verbindungen besitzen, soweit nicht Y Sauerstoff und $R^2$ mit $R^4$ identisch ist, ein optisch aktives (chirales) Zentrum am Kohlenstoff-2-atom in der Ätherseitenkette. Die Verbindungen liegen als Racemate (DL-Form) vor. Eine Herstellung bzw. Isolierung der optisch aktiven Antipoden (Enantiomeren) ist möglich.

Die erfindungsgemäßen Verbindungen der Formel 1 besitzen wertvolle pharmazeutische Eigenschaften. Sie sind in vitro und in vivo gegen verschiedene Viren wirksam und eignen sich daher zur Bekämpfung verschiedener, durch DNS-Viren verursachter Krankheiten, wie Herpes, z. B. Typus simplex 1 und 2, Cytomegalie und RNS-Viren, z. B. Retroviren, sowie Autoimmunkrankheiten und zur Behandlung von Krebs. Arzneimittel, die sie enthalten, können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0,1-10, vorzugsweise 0,2-8 mg/kg Körpergewicht verabreicht. Sie werden zweckmäßig in Dosierungseinheiten verabreicht, die mindestens die wirksame Tagesmenge der erfindungsgemäßen Verbindungen, z.B. 30-300, vorzugsweise 50-250 mg enthalten.

Die erfindungsgemäßen Verbindungen können auch in Kombination mit anderen Antivirusmitteln und Immunstimulantien, wie Interferonen verabreicht werden.

Die Wirkung erfindungsgemäßer Verbindungen wurde wie folgt geprüft.

A. In vitro-Versuche:

Zellkulturen, z.B. Hela- und Vero-Zellen, wurden in Mikrotiterplatten eingesät und mit Viren, z.B. Herpes simplex 1 infiziert. 2 Stunden nach der Infektion wurden die zu prüfenden Verbindungen den infizierten Zellkulturen in verschiedenen Verdünnungen zugegeben. 48-72 Stunden nach der Infektion wurde der Therapieerfolg anhand des cytopathogenen Effektes mikroskopisch und nach Neutralrotaufnahmen photometrisch bestimmt.

B. In vivo-Versuche:

NMRI-Mäuse, spezifisch pathogenfrei, im Gewicht von etwa 15 g wurden intraperitoneal mit Herpes simplex Typ 1 infiziert und anschließend mit den beschriebenen Verbindungen intraperitoneal therapiert. Als Vergleich diente stets Acyclovir (ACV). Die Behandlung erfolgte zweimal täglich über 5 Tage, beginnend nach der Infektion. Der Behandlungserfolg wurde anhand des Krankheitsverlaufes und der Überlebensrate gegenüber den unbehandelten Infektionskontrollen bestimmt. Letztere erhielten anstelle der zu prüfenden Verbindungen eine wasserlösliche Methylhydroxyäthylcellulose (Viskosität 300 Pa•s in 2 %iger Lösung) ((R)Tylose MH 300 P) appliziert.

Die chemaotherapeutische Wirkung erfindungsgemäßer Verbindungen ist aus Tabelle 1 ersichtlich.

Beispiele

1) Erfindungsgemäße Produkte:

1a) 9-(1-Chlormethyl-2-acetoxyäthoxymethyl)-guanin (Verbindung 6)

36.73 g (0,1 Mol) 2,6,9-Tris-(trimethylsilyl)-guanin, Rohprodukt (hergestellt nach US-Patent 4.287.188), werden in 100 ml Toluol gelöst, 20 ml Triäthylamin zugegeben und 22,0 g (0,1 Mol) + 10 % Überschuß) 1-Chlor-2-chlormethoxy-3-acetoxy-propan, gelöst in etwas Toluol, bei Raumtemperatur unter Rühren und unter Stickstoff-Schutzgas zugetropft. Die Reaktionsmischung wird sodann 20 Stunden unter Rühren und unter Stickstoff auf 90 °C erhitzt. Danach wird das Lösungsmittel Toluol abgedampft, der Rückstand in 500 ml Methanol gelöst und 30 Minuten unter Rückfluß erhitzt. Die Lösung wird nach Kohlenzusatz warm abgesaugt, eingedampft und der Rückstand zweimal aus Wasser umkristallisiert. Man erhält 16 g = 50 % d. Theorie; weiße Kristalle; Fp. 166 °C.

1b) 9-(2-Chlormethyl-2-hydroxyäthoxymethyl)-guanin (Verbindung 2)

31,55 g (0,1 Mol) der nach 1a erhaltenen Verbindung werden in eine Lösung von 500 ml flüssigem Ammonaik in 500 ml Methanol eingetragen. Die Lösung wird 12 Stunden bei Raumtemperatur gerührt, nach Zusatz von etwas Aktivkohle abgesaugt, eingedampft und der Rückstand zweimal aus wenig Wasser umkristallisiert. Man erhält 19 g = 70 % d. Theorie; weiße Kristalle; Fp. 178 °C.

Ausgangsstoffe

1c) 1-Chlor-2-chlormethoxy-3-acetoxy-propan

15,25 g (0,1 Mol) 1-Chlor-2-hydroxy-3-acetoxy-propan werden in 70 ml Methylenchlorid gelöst, 3 g (0,1 Mol) Paraformaldehyd zugegeben und unter Rühren und Eis/Wasser-Kühlung bei ca. 5 °C 2 Stunden lang Chlorwasserstoffgas eingeleitet. Nach Zugabe von 10 g Magnesiumsulfat (wasserfrei) als wasserbindendem Mittel wird nochmals 4 Stunden bei 5 °C Chlorwasserstoffgas eingeleitet. Sodann wird das Magnesiumsulfat abgesaugt und das Lösungsmittel bei möglichst niedriger Temperatur abgedampft. Der flüssige Rückstand wird direkt weiter umgesetzt. Man erhält 17 g = 85 % d. Theorie; Flüssigkeit; zersetzlich bei der Destillation.

1d) 1-Chlor-2-hydroxy-3-acetoxy-propan

Die als Ausgangsstoff verwendete Verbindung wurde nach Liebig's Annalen 402, 137 (1914) durch Umsetzung von Epichlorhydrin mit Eisessig in Gegenwart von Eisen-(III)-chlorid bei Raumtemperatur dargestellt. Flüssigkeit; Kp. 17 mm 118 °C.

2) Erfindungsgemäße Produkte

2a) 9-(1-Azidomethyl-2-acetoxyäthoxymethyl)-guanin (Verbindung 9)

36,73 g (0,1 Mol) 2,6,9-Tris-(trimethylsilyl)-guanin, Rohprodukt werden in 100 ml Toluol gelöst, 20 ml Triäthylamin zugegeben und 22,8 (0,1 Mol + 10 % Überschuß) 1-Azido-2-chlormethoxy-3-acetoxypropan, gelöst in etwas Toluol, zunächst bei Raumtemperatur,unter Rühren und unter Stickstoff-Schutzgas zugetropft. Die Reaktionsmischung wird sodann 20 Stunden unter Rühren und unter Stickstoff auf 80 °C erhitzt. Danach wird das Toluol abgedampft, der Rückstand in 500 ml Methanol gelöst und 30 Minuten unter Rückfluß erhitzt. Die Lösung wird nach Kohlezusatz warm abgesaugt, eingedampft, der Rückstand in einer Mischung von Methylenchlorid/Methanol 5:1 (Volumenverhältnis) gelöst und über eine Chromatographie-Säule, beladen mit Kieselgel (Grace Matrex L C 60 A) gereinigt. Das Eluat wird eingedampft, der Rückstand mit Äthanol angerührt, abgesaugt und mit wenig Äthanol und Äther gewaschen. Man erhält 13,7 g = 38 % der Theorie; weiße Kristalle, Fp. 198 °C.

2b) 9-(1-Aminomethyl-2-acetoxyäthoxymethyl)-guanin (Verbindung 10)

Die nach 2a) erhaltene Verbindung wird katalytisch mit Raney-Nickel/Wasserstoff in Äthylenglykolmonomethyläther bei 40 °C reduziert. Weiße Kristalle; Fp. 144 °C.

Ausgangsstoffe

2c) 1-Azido-2-chlormethoxy-3-acetoxy-propan

15,9 g (0,1 Mol) 1-Azido-2-hydroxy-3-acetoxy-propan werden in 100 ml Methylenchlorid gelöst, 3 g (0,1 Mol) Paraformaldehyd zugegeben und unter Rühren und Eis/Wasser-Kühlung bei 0-5 °C 2 Stunden lang Chlorwasserstoffgas eingeleitet. Nach Zugabe von 10 g Natriumsulfat (wasserfrei) wird weitere 4 Stunden bei 5 °C Chlorwasserstoffgas eingeleitet. Sodann wird vom Natriumsulfat abgesaugt und das Lösungsmittel bei möglichst niedriger Temperatur abgedampft. Der flüssige Rückstand wird direkt weiter umgesetzt. Man erhält 1-Azido-2-chlormethoxy-3-acetoxy-propan. Dieses wird als Rohprodukt für die Herstellung der Verbindung 9 gemäß Beispiel 2a verwendet.

2d) 1-Azido-2-hydroxy-3-acetoxy-propan

13,55 g (0,1 Mol) 1-Azido-2-hydroxy-3-chlorpropan werden mit 10,7 g (0,1 Mol + 10 % Überschuß), Kaliumacetat wasserfrei, versetzt und 1 Stunde unter Rühren auf 150 °C erhitzt. Nach dem Erkalten wird auf 200 ml Eis/Wasser gegossen und 3 Mal mit Äther ausgeschüttelt. Die Ätherextrakte werden über Natriumsulfat getrocknet, eingedampft und der Rückstand unter Vakuum der Wasserstrahlpumpe destilliert. Man erhält die Verbindung als gelbliches Öl; $Kp_1$ 80 °C.

2e) 1-Azido-2-hydroxy-3-chlor-propan

Diese Verbindung wurde nach J.Org.Chem. 21, 373 (1956) durch Umsetzung von 12,0 g (0,13 Mol) Epichlorhydrin mit 11,7 g (0.18 Mol) Natriumazid in Gegenwart von 17,9 g (0,08 Mol) Magnesiumperchlorat in Wasser bei 0 bis 5 °C hergestellt. Die Verbindung ist eine farblose Flüssigkeit vom $Kp_1$ 62 °C.

Erfindungsgemäße Produkte

3a) 9-(1-Isopropoxymethyl-2-acetoxyäthoxymethyl)-guanin (Verbindung 20)

36,73 g (0,1 Mol) 2,6,9-Tris-(trimethylsilyl)-guanin, Rohprodukt (hergestellt nach US-Patent 4.287.188), werden in 100 ml Toluol gelöst, 20 ml Triäthylamin zugegeben und 24,6 g (0,1 Mol + 10 % Überschuß) 1-isopropoxy-2-chlormethoxy-3-acetoxy-propan, gelöst in etwa Toluol, bei Raumtemperatur unter Rühren und unter Stickstoff-Schutzgas zugetropft. Die Reaktionsmischung wird zunächst, wie im Beispiel 2 beschrieben, behandelt und eingedampft. Der eingedampfte Rückstand enthält je nach Ansatz die 7- und 9-Isomeren in wechselnden Mengenverhältnissen. Die Schmelzpunkte schwanken daher zwischen 209 und 225 °C. Eine Trennung dieser Isomeren ist über eine Büchi-Chromatographiesäule, gefüllt mit Grace-Kieselgel (R)Matrex LC 60A und der Laufmittelmischung Methylenchlorid/Cyclohexan/Methanol/Eisessig (Volumenverhältnis 100:20:10:1) möglich. Nach Eindampfen des Eluats wird der Rückstand mit Wasser angerührt, abgesaugt und mit Wasser und wenig kalten Äthanol gewaschen und getrocknet. Aus einem Rückstand vom Fp. 212 °C erhäült man so 9 g =(26 % d. Theorie), weiße Kristalle vom Fp. 232 °C (95 % 9-Isomeres nach HPLC-Analyse über Merck Li-Chrosorb Si 60/5 µm).

3b) 9-(1-Isopropoxymethyl-2-hydroxyäthoxymethyl)-guanin (Verbindung 14)

33,9 g (0,1 Mol) 9-(1-Isopropoxymethyl-2-acetoxyäthoxy methyl)-guanin werden in eine Lösung von 500

ml flüssigem Ammoniak in 500 ml Methanol eingetragen. Die Lösung wird 12 Stunden bei Raumtemperatur gerührt, nach Zusatz von etwas Akativkohle abgesaugt, eingedampft und der Rückstand 2 mal aus Wasser umkristallisiert. Man erhält 17 g = 57 % der Theorie; weiße Kristalle, Fp. 191 °C.

Dieselbe Verbindung 14 kann auch nach Verfahren B wie folge hergestellt werden. Dazu werden 23,5 g (0,1 Mol) 2,9-Diacetyl-guanin zusammen mit 27,3 g (0,1 Mol + 10 % Überschuß) 1-Isopropoxy-2-acetoxymethoxy-3-acetoxypropan und 0,2 g 4-Toluolsulfonsäure in 60 ml wasserfreiem Sulfolan 60 Stunden unter Rühren auf 95 °C erhitzt. Die erkaltete Reaktionsmischung wird mit 500 ml Toluol verdünnt, filtriert und über Kieselgel chromatographiert. Die Eluation erfolgt mit einer Mischung von Methylenchlorid/Methanol (Volumenverhäntis 10:1). Das Eluat wird eingedampft, der Rückstand (2-Acetyl-7(9)-(1-Isopropoxymethyl-2-acetoxy-äthoxymethyl)-guanin-Isomerengemisch) wird in eine Lösung von 500 ml flüssigem Ammoniak in 500 ml Methanol eingetragen und 12 Stunden bei Raumtemperatur gerührt. Nach Zusatz von etwas Aktivkohle wird abgesaugt, eingedampft und der Rückstand aus Wasser umkristallisiert. Man erhält 9 g = 37 % d. Theorie, weiße Kristalle, Fp. 190 °C.

Ausgangsstoffe

3c) 1-Isopropoxy-2-chlormethoxy-3-acetoxy-propan

17,6 g (0,1 Mol) 1-Isopropoxy-2-hydroxy-3-acetoxy-propan werden mit Formaldehyd und Chlorwasserstoffgas analog Beispiel 2c) umgesetzt. Man erhält 20 g der Verbindung als Flüssigkeit (90 % der Theorie); zersetzlich bei der Destillation.

3d) 1-Isopropoxy-2-hydroxy-3-acetoxy-propan

15,25 g (0,1 Mol) 1-Isopropoxy-2-hydroxy-3-chlor-propan werden mit wasserfreiem Kaliumacetat analog Beispiel 2d) umgesetzt. Man erhält 15 g = 85 % der Theorie; Flüssigkeit; $Kp_{22}$ 128 °C.

3e) 1-Isopropoxy-2-hydroxy-3-chlor-propan

Die als Ausgangsstoff verwendete Verbindung wurde nach J.Org.Chem. 12, 831 (1947) durch Umsetzung von Epichlorhydrin mit Isopropanol in Gegenwart von konzentrierter Schwefelsäure bei Raumtemperatur dargestellt. Flüssigkeit; $Kp_{22}$ 89 °C.

3f) 2,9-Diacetyl-guanin

Diese Verbindung wurde analog Bull.Chem. Soc.Jap. 37 (9), 1389 durch Umsetzung von Guanin mit Acetanhydrid in Gegenwart von etwas konz. Schwefelsäure (30 Minuten Rückfluß) hergestellt. Fp. 265 °C.

3g) 1-Isopropoxy-2-acetoxymethoxy-3-acetoxy-propan

Diese Verbindung wurde durch Umsetzung von 1-Isopropoxy-2-chlormethoxy-3-acetoxy-propan (3c) mit überschüsigem Kaliumacetat in Aceton (12 Stunden bei Raumtemperatur) analog J.Med.Chem. 26, 759 (1983) hergestellt und als Rohprodukt direkt weiter umgesetzt.

Erfindungsgemäße Produkte

4a) 2-Amino-6-isopropoxy-9-(1-isopropoxymethyl-2-acetoxyäthoxymethyl)-purin (Verbindung 44)

19,3 g (0,1 Mol) 2-Amino-6-isopropoxy-purin und 13,2 g (0,1 Mol) Ammoniumsulfat werden in 200 ml Hexamethyldisilazan unter Stickstoff eingetragen und 4 Stunden auf 130 °C erhitzt. Nach Abkühlen wird das

überschüssige Hexamethyldisilazan unter Wasserstrahlvakuum abdestilliert, der feste Rückstand (6-Isopropyl-2,9-bis-(trimethylsilyl)-guanin) in 100 ml Toluol gelöst, 20 ml Triäthylamin zu gegeben und unter Rühren und unter Stickstoff 24,6 g (0,1 Mol + 10 % Überschuß) 1-Isopropoxy-2-chlormethoxy-3-acetoxy-propan, gelöst in etwas Toluol, bei Raumtemperatur zugetropft. Die Reaktionsmischung wird sodann 12 Stunden unter Rühren und Stickstoff auf 90 °C erhitzt. Danach wird das Toluol abgedampft, der Rückstand in 200 ml Methanol gelöst und 20 Minuten unter Rückfluß erhitzt. Das Methanol wird abgedampft, der Rückstand in Methylenchlorid/Methanol (Volumenverhältnis 40:1) gelöst und über eine Chromatographiesäule, beladen mit Kieselgel (Fa. Grace, Matrex LC 60A gereinigt. Das Eluat wird eingedampft, der ölige Rückstand durch Anrühren mit Essigsäureäthylester/Cyclohexan (Volumenverhältnis 2:1) zur Kristallisation gebracht. Die Kristalle werden abgesaugt und mit Essigsäureäthylester/Diäthyläther gewaschen und im Exsikkator getrocknet. Man erhält 19 g = 50 % der Theorie; weiße Kristalle; Fp. 137 °C (98 % 9-Isomeres nach HPLC-Analyse über Merck Li-Chrosorb Si 60/5 µm).

Ausgangsstoffe

4b) 2-Amino-6-isopropoxy-purin

Diese Verbindung wurde durch Umsetzung von 2-Amino-6-chlor-purin mit Natriumisopropylat in Isopropanol (12-stündiges Kochen unter Rückfluß) analog J.Org.Chem. 25, 1573 (1960) in hohen Ausbeuten hergestellt. Fp. 206 °C.

Erfindungsgemäße Produkte

5a) 9-(1,3-Bis-(isopropoxy)-propyl-2-oxymethyl)-guanin (bekannt aus der EP-A-187 297) Verbindung 49

36,73 g (0,1 Mol) 2,6,9-Tris-(trimethylsilyl)-guanin, Rohprodukt, werden mit 24,7 g (0,1 Mol + 10 % Überschuß) 1,3-Bis-(isopropoxy)-2-chlormethoxy-propan in Gegenwart von 20 ml Triäthylamin in Toluol wie unter Beispiel 2b) umgesetzt. Danach wird das Toluol abgedampft, der Rückstand in 200 ml Methanol gelöst und 20 Minuten unter Rückfluß erhitzt. Das Methanol wird abgedampft, der Rückstand in Methylenchlorid/Methanol (Volumenverhältnis 9:1) gelöst und über Kieselgel Fa. Grace, Matrex LC 60A chromatographiert. Das Eluat wird eingedampft, der kristalline Rückstand aus Äthanol umkristallisiert. Man erhält 9,5 g = 42% der Theorie; weiße Kristalle; Fp. 222 °C (96 % 9-Isomeres nach HPLC-Analyse über Merck Li-Chrosorb Si 60/5 µm).

Ausgangsstoffe

5b) 1,3-Bis-(isopropoxy)-2-chlormethoxy-propan

17,6 g (0,1 Mol) 1,3-Bis-(isopropoxy)-2-hydroxy-propan wurden mit 3 g (0,1 Mol) Formaldehyd und Chlorwasserstoffgas bei 0 bis 5 °C hergestellt und als Rohprodukt direkt weiter umgesetzt.

5c) 1,3-Bis-(isopropoxy)-2-hydroxy-propan

11,6 g (0,1 Mol) 2,3-Epoxypropyl-isopropyläther wurde mit Isopropanol in Gegenwart von katalytischen Mengen Zinn-IV-chlorid zunächst bei Raumtemperatur und danach bei langsam auf 80 °C ansteigender Temperatur umgesetzt. Farblose Flüssigkeit; Kp 9 mm 80 °C.

6 ff)

Nach den unter 1 bis 5) beschriebenen Verfahren lassen sich die in der Tabelle 2 aufgeführten

Verbindungen herstellen. Diese Verbindungen sind dort außer durch ihre Schmelzpunkte durch die NMR-Spektren identifiziert. Verbindung 40 ist 2-Amino-9-(1-isopropoxymethyl-2-acetoxyäthoxymethyl)-purin, Verbindung 48 ist das 2-Amino-9-[1,3-bis-(isopropoxy)-propyl-2-oxymethyl]-purin und Verbindung 52 ist das 2-Amino-6-isopropoxy-9-[1,3-bis-(isopropoxy)-propyl-2-oxymethyl]-purin.

Tabelle 1

| Herpes simplex Typ 1 an der NMRI-Maus | | | | |
|---|---|---|---|---|
| Präparat No. | Dosierung mg/Maus i.p. | Mittlere Überlebensrate x/10 | Dosierung mg/Maus i.p. | Mittlere Überlebensrate x/10 |
| 6 | 10 x 0,6 | 9/10 | 10 x 0,2 | 8/10 |
| 7 | 10 x 0,6 | 9/10 | 10 x 0,2 | 8/10 |
| 9 | 10 x 0,6 | 9/10 | 10 x 0,2 | 9/10 |
| 14 | 10 x 0,6 | 10/10 | 10 x 0,2 | 8/10 |
| 20 | 10 x 0,6 | 10/10 | 10 x 0,2 | 10/10 |
| 22 | 10 x 0,6 | 10/10 | 10 x 0,2 | 9/10 |
| 24 | 10 x 0,6 | 10/10 | 10 x 0,2 | 8/10 |
| 28 | 10 x 0,6 | 10/10 | 10 x 0,2 | 8/10 |
| 29 | 10 x 0,6 | 10/10 | 10 x 0,2 | 8/10 |
| 30 | 10 x 0,6 | 10/10 | 10 x 0,2 | 8/10 |
| 31 | 10 x 0,6 | 9/10 | 10 x 0,2 | 7/10 |
| 39 | 10 x 0,6 | 10/10 | 10 x 0,2 | 8/10 |
| 40 | 10 x 0,6 | 10/10 | 10 x 0,2 | 8/10 |
| 42 | 10 x 0,6 | 10/10 | 10 x 0,2 | 8/10 |
| 44 | 10 x 0,6 | 10/10 | 10 x 0,2 | 9/10 |
| 48 | 10 x 0,6 | 10/10 | 10 x 0,2 | 9/10 |
| 49 | 10 x 0,6 | 10/10 | 10 x 0,2 | 9/10 |
| 52 | 10 x 0,6 | 10/10 | 10 x 0,2 | 9/10 |
| Acyclovir | | 9/10 | | 8/10 |
| Infektionskontrollen | 10 x 0,5 ml Tylose | 2/10 | 10 x 0,5 ml Tylose® | 2/10 |
| Acyclovir = 9-(2-hydroxyäthoxymethyl)-guanin | | | | |

Tabelle 2

| No. | Formel I $R^1$ | $R^2$ | X | $R^3$ | Fp. °C | s,2H $NH_2$ C-2 | s,1H C-6 | s,1H CH C-8 | s,2H $NCH_2X$ | m,1H XCH< | m,2H $CH_2OH^2$ | s,3H $OCOCH_3$ | m,2H $CH_2R^3$ | m $R^3$ |
|---|---|---|---|---|---|---|---|---|---|---|---|---|---|---|
| | | | | | | NMR δ-Werte in Dimethylsulfoxyd ($Me_2SO-d_6$, 270 MHz) | | | | | | | | |
| 1 | OH | H | O | F | 180 | 6,45 | 10,6 | 7,8 | 5,3 | 4,15 | 3,7 | - | 4,1 | |
| 2 | | | | Cl | 178 | 6,45 | 10,6 | 7,8 | 5,3 | 4,15 | 3,7 | - | 4,1 | |
| 3 | | | | Br | 158 | 6,45 | 10,6 | 7,8 | 5,3 | 4,15 | 3,7 | - | 4,1 | |
| 4 | | | | J | 150 | 6,45 | 10,6 | 7,8 | 5,3 | 4,15 | 3,7 | - | 4,1 | |
| 5 | OH | Ac | O | F | 171 | 6,45 | 10,6 | 7,8 | 5,4 | 4,15 | 3,6 | 2,0 | 4,1 | |
| 6 | | | | Cl | 166 | 6,45 | 10,6 | 7,8 | 5,4 | 4,15 | 3,7 | 2,0 | 4,1 | |
| 7 | | | | Br | 151 | 6,45 | 10,6 | 7,8 | 5,4 | 4,15 | 3,7 | 2,0 | 4,0 | |
| 8 | | | | J | 145 | 6,4 | 10,55 | 7,8 | 5,4 | 4,15 | 3,75 | 2,0 | 4,1 | |
| 9 | | | | $N_3$ | 198 | 6,45 | 10,6 | 7,8 | 5,4 | 4,15 | 3,7 | 2,0 | 4,0 | |
| 10 | | | | $NH_2$ | 144 | 6,45 | 10,6 | 7,8 | 5,4 | 4,15 | 3,1 | 2,0 | 3,45 | |
| | | | | | | | | | s,2H $NCH_2O$ | m,1H $OCH(CH_2)_2$ | m,2H $CH_2OAc$ | | | |
| 14 | OH | H | O | $OCH(CH_3)_2$ | 196 | 6,45 | 10,6 | 7,8 | 5,3 | 4,15 | 3,5 | - | 4,0 | $OCH(CH_3)_2$ 3,35/1,02 |
| 16 | | | | $OCH(CH_3)C_2H_5$ | 200 | 6,45 | 10,6 | 7,8 | 5,3 | 4,15 | 3,5 | - | 4,0 | $OCH(CH_3)C_2H_5$ 3,4/1,0 |

Tabelle 2, Forts.

| | Formel I | | | | | NMR δ-Werte in Dimethylsulfoxyd ($Me_2SO-d_6$, 270 MHz) | | | | | | | | | |
|---|---|---|---|---|---|---|---|---|---|---|---|---|---|---|---|
| No. | $R^1$ | $R^2$ | X | $R^3$ | Fp. °C | s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m $R^3$ | |
| 17 | OH | Ac | O | $OCH_3$ | 214 | 6,4 | 10,55 | 7,8 | 5,3 | 4,15 | 3,6 | 1,9 | 4,0 | $OCH_3$ | 3,15 |
| 18 | | | | $OC_2H_5$ | 220 | 6,45 | 10,6 | 7,75 | 5,3 | 4,15 | 3,6 | 1,9 | 4,0 | $OC_2H_5$ | 3,3/1,0 |
| 19 | | | | $O(CH_2)_2CH_3$ | 216 | 6,45 | 10,6 | 7,8 | 5,3 | 4,15 | 3,6 | 1,9 | 4,0 | $O(CH_2)_2CH_3$ | 1,4/0,8 |
| 20 | | | | $OCH(CH_3)_2$ | 232 | 6,45 | 10,6 | 7,7 | 5,35 | 4,15 | 3,55 | 1,9 | 3,9 | $OCH(CH_3)_2$ | 3,35/1,0 |
| 21 | | | | $O(CH_2)_3CH_3$ | 213 | 6,45 | 10,6 | 7,8 | 5,3 | 4,15 | 3,6 | 1,9 | 3,9 | $O(CH_2)_3CH_3$ | 1,5/0,8 |
| 22 | | | | $OCH(CH_3)C_2H_5$ | 219 | 6,45 | 10,55 | 7,8 | 5,3 | 4,15 | 3,55 | 1,9 | 3,9 | $OCH(CH_3)C_2H_5$ | 3,4/1,0 |
| 23 | | | | $OCH_2CH(CH_3)_2$ | 217 | 6,45 | 10,6 | 7,8 | 5,35 | 4,15 | 3,6 | 2,0 | 4,0 | $CH_3$<br>$OCH_2CH$<br>$CH_3$ | 3,1<br>3,4<br>0,8 |
| 24. | | | | $OC(CH_3)_3$ | 185 | 6,45 | 10,6 | 7,8 | 5,3 | 4,15 | 3,6 | 1,9 | 4,0 | $OC(CH_3)_3$ | 1,1 |
| 25 | | | | $O(CH_2)_4CH_3$ | 210 | 6,45 | 10,6 | 7,8 | 5,3 | 4,15 | 3,6 | 1,9 | 4,0 | $O(CH_2)_4CH_3$ | 1,7/0,8 |
| 26 | | | | $OCH(C_2H_5)_2$ | 221 | 6,45 | 10,6 | 7,8 | 5,35 | 4,15 | 3,45 | 1,85 | 4,0 | $C_2H_5$<br>$OCH$<br>$C_2H_5$ | 3,4<br>1,2<br>0,8 |
| 27 | | | | O 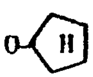 | 212 | 6,5 | 10,6 | 7,8 | 5,4 | 4,15 | 3,6 | 1,9 | 4,0 | O 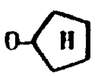 | 1,5 |

Tabelle 2, Forts.

| No. | $R^1$ | $R^2$ | X | $R^3$ | Fp. °C | s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m $R^3$ | |
|---|---|---|---|---|---|---|---|---|---|---|---|---|---|---|---|
| 28 | OH | Ac | O | $OCH_2CH{=}CH_2$ | 209 | 6,4 | 10,6 | 7,8 | 5,3 | 4,15 | 3,4 | 1,95 | 3,9 | $OCH_2CH{=}CH_2$ | 5,0-6,0 |
| 29 | | | | $OCH_2C_6H_5$ | 186 | 6,5 | 10,65 | 7,8 | 5,35 | 4,15 | 3,4 | 1,95 | 4,0 | $OCH_2{-}C_6H_5$<br>$-C_6H_5$ | 4,45<br>7,3 |
| 30 | | | | $OCH(CH_3)C_6H_5$ | 180 | 6,45 | 10,6 | 7,7 | 5,35 | 4,15 | 3,55 | 1,85 | 4,0 | $CH_3$<br>OCH<br>$C_6H_5$ | 1,25<br>4,4<br>7,3 |
| 31 | | | | $OCH_2CF_3$ | 208 | 6,45 | 10,6 | 7,8 | 5,3 | 4,15 | 3,6 | 1,95 | 3,9 | $OCH_2CF_3$ | 3,2 |

NMR δ-Werte in Dimethylsulfoxyd ($Me_2SO\text{-}d_6$, 270 MHz); Formel I: $R^1$, $R^2$, X, $R^3$

Tabelle 2, Forts.

NMR δ-Werte in Dimethylsulfoxyd ($Me_2SO-d_6$, 270 MHz)

| Formel I | | | | | | | | | | | | | | | |
|---|---|---|---|---|---|---|---|---|---|---|---|---|---|---|---|
| No. | $R^1$ | $R^2$ | X | $R^3$ | Fp. °C | s,2H $NH_2$ C-2 | s,1H C-6 | s,1H CH C-8 | s,2H $NCH_2X$ | m,1H $XCH(CH_2)_2$ | m,2H $CH_2OR^2$ | $OR^2$ | m,2H $CH_2R^3$ | m,1H $CH(CH_3)_2$ | d,6H $CH(CH_3)$ |
| 32 | OH | H | O | $SCH(CH_3)_2$ | 187 | 6,45 | 10,6 | 7,8 | 5,35 | 4,15 | 2,8 | - | 3,5 | 3,2 | 1,1 |
| 33 | | | | $SOCH(CH_3)_2$ | 118 | 6,45 | 10,6 | 7,8 | 5,4 | 4,15 | 2,8 | - | 3,7 | 3,4 | 1,1 |
| 34 | | | | $SO_2CH(CH_3)_2$ | 146 | 6,45 | 10,6 | 7,8 | 5,4 | 4,15 | 2,8 | - | 3,8 | 3,5 | 1,1 |
| 35 | OH | Ac | O | $SCH(CH_3)_2$ | 198 | 6,45 | 10,6 | 7,8 | 5,4 | 4,15 | 3,6 | 1,9 | 4,0 | 3,2 | 1,2 |
| 36 | | | | $SOCH(CH_3)_2$ | 127 | 6,45 | 10,6 | 7,8 | 5,4 | 4,3 | 3,7 | 1,9 | 4,2 | 3,4 | 1,2 |
| 37 | | | | $SO_2CH(CH_3)_2$ | 157 | 6,45 | 10,6 | 7,8 | 5,4 | 4,3 | 3,7 | 1,9 | 4,3 | 3,5 | 1,2 |
| 38 | OH | H | S | $OCH(CH_3)_2$ | 196 | 6,4 | 10,5 | 7,7 | 5,1 | 4,15 | 2,7 | - | 3,5 | 3,35 | 1,1 |
| 39 | OH | Ac | S | $OCH(CH_3)_2$ | 205 | 6,4 | 10,5 | 7,7 | 5,1 | 4,15 | 3,6 | 1,9 | 3,35 | 3,35 | 1,1 |
| | | | | | | | s,1-2H C-6 | | s,2H $NCH_2O$ | m,1H $OCH(CH_2)_2$ | | s,3H $OCOCH_3$ | m,2H $CH_2OC_3H_7$ | | |
| 40 | H | Ac | O | $OCH(CH_3)_2$ | 89 | 5,53 | 8,72 | 7,88 | 5,13 | 4,3 | 3,5 | 2,05 | 3,72 | 3,4 | 1,08 |
| 41 | Cl | Ac | O | " | 122 | 4,8 | - | 7,9 | 5,45 | 4,3 | 3,5 | 2,1 | 3,7 | 3,4 | 1,05 |
| 42 | $NH_2$ | Ac | O | " | 168 | 6,68 | 7,75 | 8,02 | 5,38 | 4,15 | 3,6 | 1,98 | 3,9 | 3,4 | 1,0 |
| 43 | SH | Ac | O | " | 192 | 6,9 | 13,1 | 8,2 | 5,35 | 4,15 | 3,5 | 1,85 | 3,9 | 3,4 | 1,0 |

**Tabelle 2, Forts.**

| No. | Formel I $R^1$ | $R^2$ | X | $R^3$ | Fp. °C | NMR δ-Werte in Dimethylsulfoxyd ($Me_2SO-d_6$, 270 MHz) s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m,1H $OCH(CH_3)_2$ | d,6H $OCH(CH_3)_2$ |
|---|---|---|---|---|---|---|---|---|---|---|---|---|---|---|---|
| 44 | $OCH(CH_3)_2$ | Ac | O | $OCH(CH_3)_2$ | 137 | 6,4 | s,1H $OCH(CH_3)_2$ 5,3 d,6H $OCH(CH_3)_2$ 1,35 | 8,0 | 5,5 | 4,15 | 3,4 | 1,8 | 4,0 | 3,6 | 1,0 |
| | | | | | | | | | | | | | | m $R^3$ | |
| 45 | $OCH_3$ | Ac | O | $OCH(CH_3)_2$ | 96 | 6,4 | - | 7,9 | 5,4 | 4,0 | 3,4 | 1,9 | 3,9 | $OCH(CH_3)_2$ | 3,35/1,0 |
| 46 | 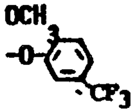 | Ac | O | $OCH(CH_3)_2$ | Öl | 5,0 | 7,4 | 7,8 | 5,5 | 4,1 | 3,7 | 2,0 | 3,7 | $OCH(CH_3)_2$ | 3,35/1,1 |
| 47 | $N_3$ | Ac | O | $OCH(CH_3)_2$ | 116 | 8,4 | - | 8,3 | 5,6 | 4,1 | 3,4 | 1,9 | 3,7 | $OCH(CH_3)_2$ | 3,35/0,9 |
| 48 | H | $-CH(CH_3)_2$ | O | $OCH(CH_3)_2$ | 81 | 6,5 | 8,7 | 8,2 | 5,6 | 3,8 | 3,3 | - | 3,5 | $2OCH(CH_3)_2$ | 3,35/1,0 |
| | | | | | | | | | | | | | | m2(1H) $OCH(CH_2)_2$ | d2(6H) $OCH(CH_2)$ |
| 49 | OH | $-CH(CH_3)_2$ | O | $OCH(CH_3)_2$ | 222 | 6,4 | 10,55 | 7,75 | 5,35 | 4,15 | | | | 3,6 | 1,0 |

**Tabelle 2, Forts.**

| No. | Formel I $R^1$ | $R^2$ | X | $R^3$ | Fp. °C | NMR δ-Werte in Dimethylsulfoxyd ($Me_2SO-d_6$, 270 MHz) s,2H $NH_2$ C-2 | s,1H C-6 | s,1H CH C-8 | s,2H $NCH_2O$ | m,1H $OCH(CH_2)_2$ | m,2H $CH_2OAc$ | s,3H $OCOCH_3$ | m,2H $CH_2R^3$ | m,1H $OCH(CH_3)_2$ | d,6H $OCH(CH_3)_2$ |
|---|---|---|---|---|---|---|---|---|---|---|---|---|---|---|---|
| | | | | | | | | | | | m,4H $CH_2OR^{2+3}$ | | m $R^3$ | | |
| 50 | OH | $-CH_2-CH=CH_2$ | O | $-OCH_2CH=CH_2$ | 216 | 6,45 | 10,65 | 7,8 | 5,4 | 4,1 | 3,9/3,4 | | $2OCH_2CH=CH_2$ | | 5,0-6,0 |
| | | | | | | | | | | | | | m,4H | | |
| 51 | OH | $-CH_2CF_3$ | O | $-OCH_2CF_3$ | 212 | 6,4 | 10,6 | 7,8 | 5,4 | 4,3 | 3,7 | | $2OCH_2CF_3$ | | 4,0 |
| | | | | | | | | | | | | | m $R^3$ | | |
| 52 | $-OCH(CH_3)_2$ | $-CH(CH_3)_2$ | O | $-OCH(CH_3)_2$ | ~100 | 6,35 | - | 7,95 | 5,6 | 4,1 | 3,8/3,4 | | $3OCH(CH_3)_2$ | | 3,35/1,0 |

**Ansprüche**

1. 2-Amino-9-[1,3-bis-(isopropoxy)-propyl-2-oxymethyl]-purin, eine Verbindung der Formel

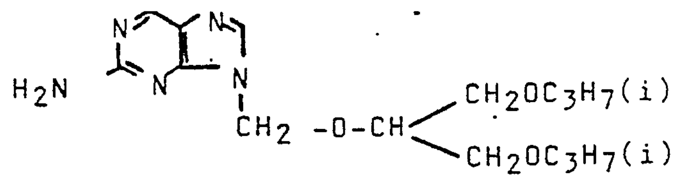

2. Arzneimittel, gekennzeichnet durch einen Gehalt an der Verbindung gemäß Anspruch 1.
3. Verwendung der Verbindung gemäß Anspruch 1 zur Herstellung von Arzneimitteln zur Behandlung von durch Viren verursachten Krankheiten.

Patentansprüche für folgenden Vertragsstaat: AT

1. Verfahren zur Herstellung von Verbindungen der Formel 1

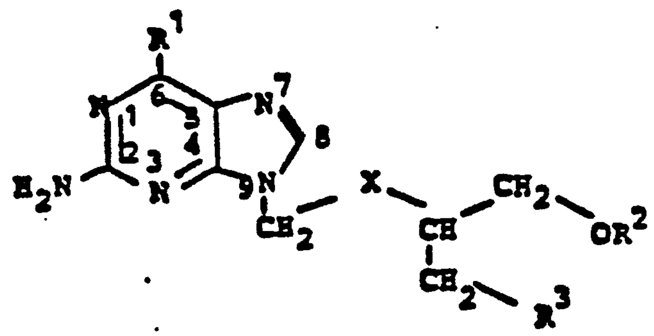

1

worin
$R^1$ H, Halogen, OH, SH, Azido, $NH_2$ oder Z-$R^5$ ist, wobei bedeuten Z O, S, SO, $SO_2$ oder NH und $R^5$ einen acyclischen aliphatischen Kohlenwasserstoffrest mit 1 bis 6 C-Atomen, welcher a) unsubstituiert ist oder b) durch wenigstens eines der Merkmale modifiziert ist, daß er eine Alkoxygruppe mit 1 bis 3 C-Atomen oder eine Doppelbindung enthält oder mindestens teilweise fluoriert ist, oder Cycloalkyl mit 3 bis 6 C-Atomen oder Phenylalkyl, das direkt an Z oder über eine Alkylengruppe von 1 bis 3 C-Atomen gebunden ist, oder Phenyl, wobei der Phenylring jeweils höchstens zweifach, vorzugsweise höchstens einmal durch einen Substituenten aus der Gruppe Halogen, Trifluormethyl, Alkoxy und Alkylthio mit jeweils 1 bis 3 C-Atomen substituiert sein kann, $R^2$ H ist oder die Bedeutung von $R^5$ hat oder eine Acylgruppe $COR^6$ ist, wobei $R^6$ Alkyl mit 1 - 6 C-Atomen, Benzyl oder Phenyl ist,
$R^3$ Halogen, Azido, unsubstituiertes Amino oder den Rest Y-$R^4$ bedeutet, wobei $R^4$ dieselble Bedeutung wie $R^5$ hat und Y ebenso wie
X O, S, SO oder $SO_2$ bedeutet und X und Y gleich oder verschieden sein können,
wobei 9-(1,3-Bis-isopropoxy)-propyl-2-oxymethyl)-guanin und solche Verbindungen ausgenommen sind, in denen nebeneinander $R^1$ OH oder SH, $R^2$ H, $R^3$ Y-$R^4$, Y O oder S, Y O und $R^4$ Alkyl mit 1 bis 6 C-Atomen sind bzw. in denen nebeneinander $R^1$ OH oder $NH_2$, $R^2$ Benzyl Y-$R^4$ mit Y=O und $R^4$ = Benzyl und X O ist, dadurch gekennzeichnet, daß man unter üblichen Bedingungen
A) zur Herstellung von Verbindungen, in denen $R^1$ Hydroxy, Amino- oder Wasserstoff ist, 2,6,9-Tris-(trialkylsilyl)- bzw. 2,9-Bis-(trialkylsilyl)-2-amino-purine gemäß einer der Formeln 8, 11 und 12

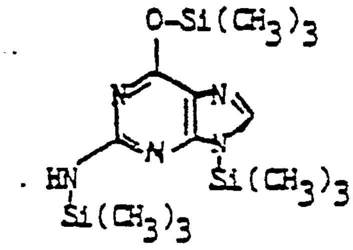

8

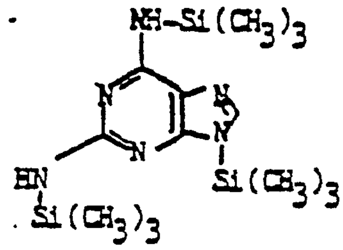

11

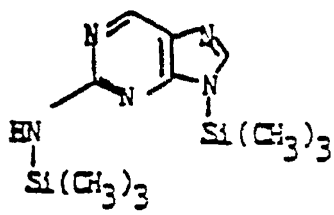

12

mit Halogen-, insbesondere Chlorverbindungen der Formel

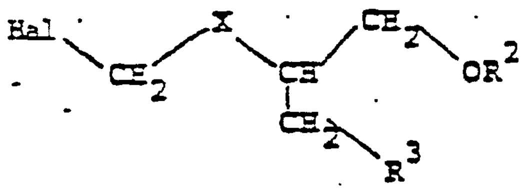

13

umsetzt, worin $R^2$ = Acyl ist und X und $R^3$ die angegebene Bedeutung haben, und die Trialkylsilylschutzgruppen abspaltet, oder

B) zur Herstellung von Verbindungen, in denen $R^1$ Hydroxy, Mercapto, Amino oder Wasserstoff ist, entsprechende D1- bzw. Triacyl-Verbindungen der Formeln 9, 10, 6a bzw. 7a

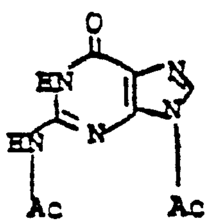

9

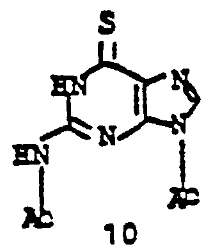

10

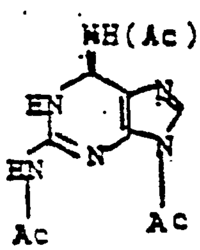

6a

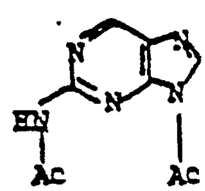

7a

mit reaktiven Acetoxy-Verbindungen der Formel 14

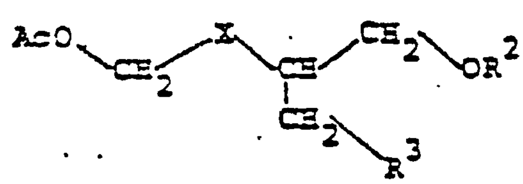

14

umsetzt, worin $R^2$ Acyl ist und X und $R^3$ die oben angegebene Bedeutung haben, oder

C) zur Herstellung von Verbindungen, in denen $R^1$ Halogen ist, Verbindungen der Formel 5

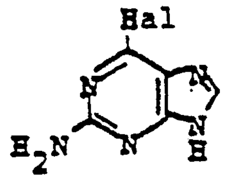

5

mit aktiven Halogen-Verbindungen der unter A angegebenen Formel 13 umsetzt und die so erhaltene Verbindung 18 isoliert oder durch Saure Verseifung zu Verbindungen, in denen $R^1$ Hydroxy ist, oder durch Umsetzung mit Schwefelwasserstoff zu Verbindungen, in denen $R^1$ Mercapto ist, oder durch Umsetzung mit Alkaliaziden zu Verbindungen, in denen $R^1$ Azido ist, oder durch Umsetzung mit Ammoniak zu Verbindungen, in denen $R^1$ Amino ist, oder durch katalytische dehalogenierende Hydrierung zu Verbindungen, in denen $R^1$ Wasserstoff ist, oder durch Umsetzung mit Alkoholen bzw. Phenolen, Mercaptanen bzw. Thiophenolen oder Aminen bzw. Anilinen zu Verbindungen, in denen $R^1$ $ZR^5$ ist, umsetzt oder

D) zur Herstellung von Verbindungen, in denen $R^2$ Wasserstoff ist, eine Verbindung, die nach einem der Verfahren A bis C hergestellt ist, verseift.

wobei die Verbindung der Formel 1 das 2-Amino-9-[1,3-bis-(isopropoxy)-propyl-2-oxymethyl]-purin ist.

2. Verfahren zur Herstellung einer pharmazeutischen Komposition, die zur Behandlung von durch Viren verursachten Krankheiten geeignet ist, dadurch gekennzeichnet, daß man als aktive Substanz die Verbindung der Formel 1 einarbeitet, die nach dem Verfahren gemäß Anspruch 1 hergestellt ist, in Kombination mit einem pharmazeutisch unbedenklichen Träger derart, daß man eine für die Verabreichung geeignete Komposition erhält.

3. Verfahren zur Herstellung von Arzneimittelformulierungen zur Behandlung von durch Viren verursachten Krankheiten, dadurch gekennzeichnet, daß man die Verbindung der Formel 1, die nach dem Verfahren gemäß Anspruch 1 erhalten worden ist, mit den üblichen Träger- und Hilfsstoffen zu Arzneimitteln formuliert.

FIG.1

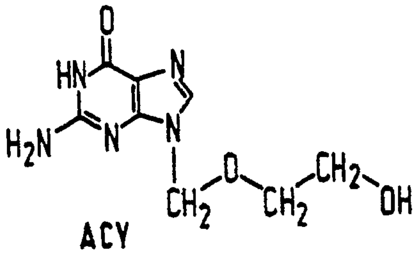

ACY

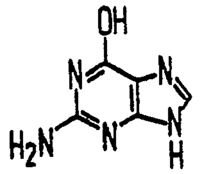

2'

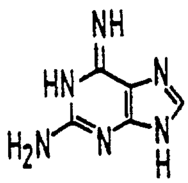

3'

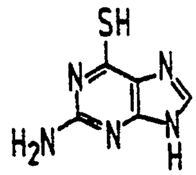

6'

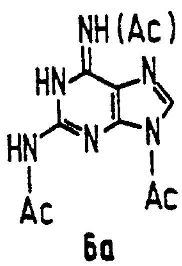

6a

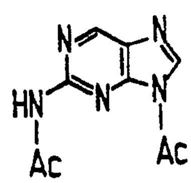

7a

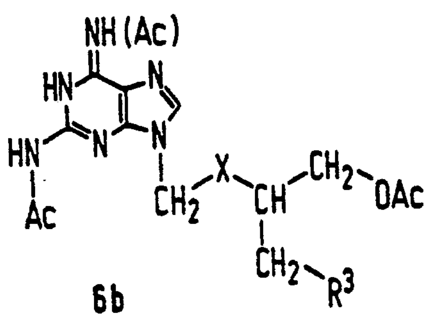

6b

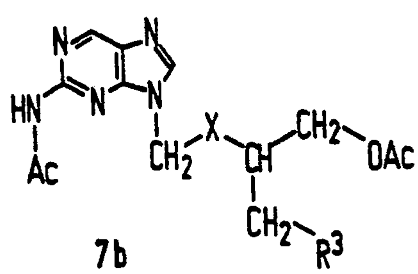

7b

FIG.2 / Teil 1

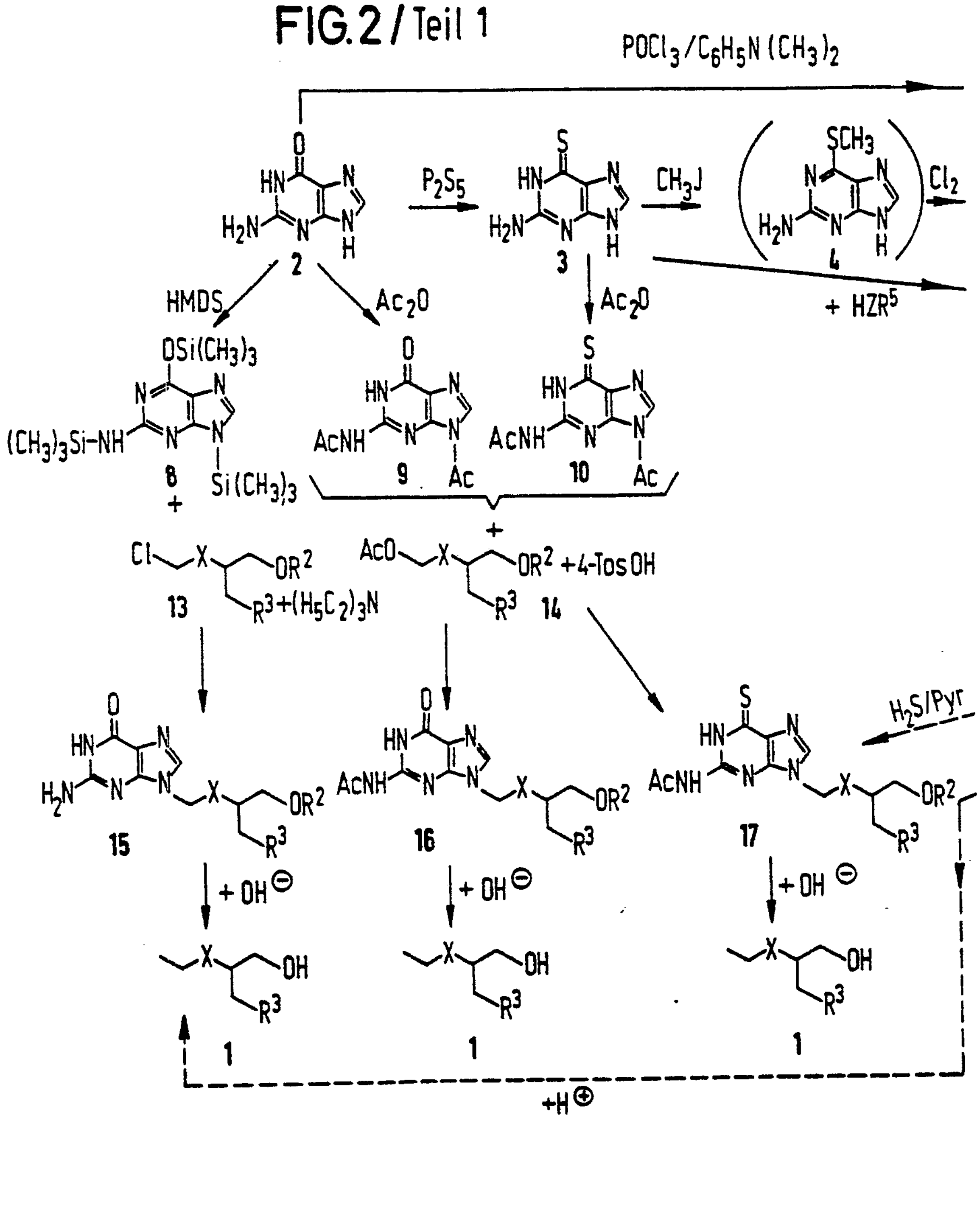
POCl3/C6H5N(CH3)2
P2S5
CH3J
Cl2
+ HZR5
HMDS
Ac2O
Ac2O
+4-TosOH
+(H5C2)3N
H2S/Pyr
+ OH⊖
+ OH⊖
+ OH⊖
+H⊕

FIG.2/Teil 2

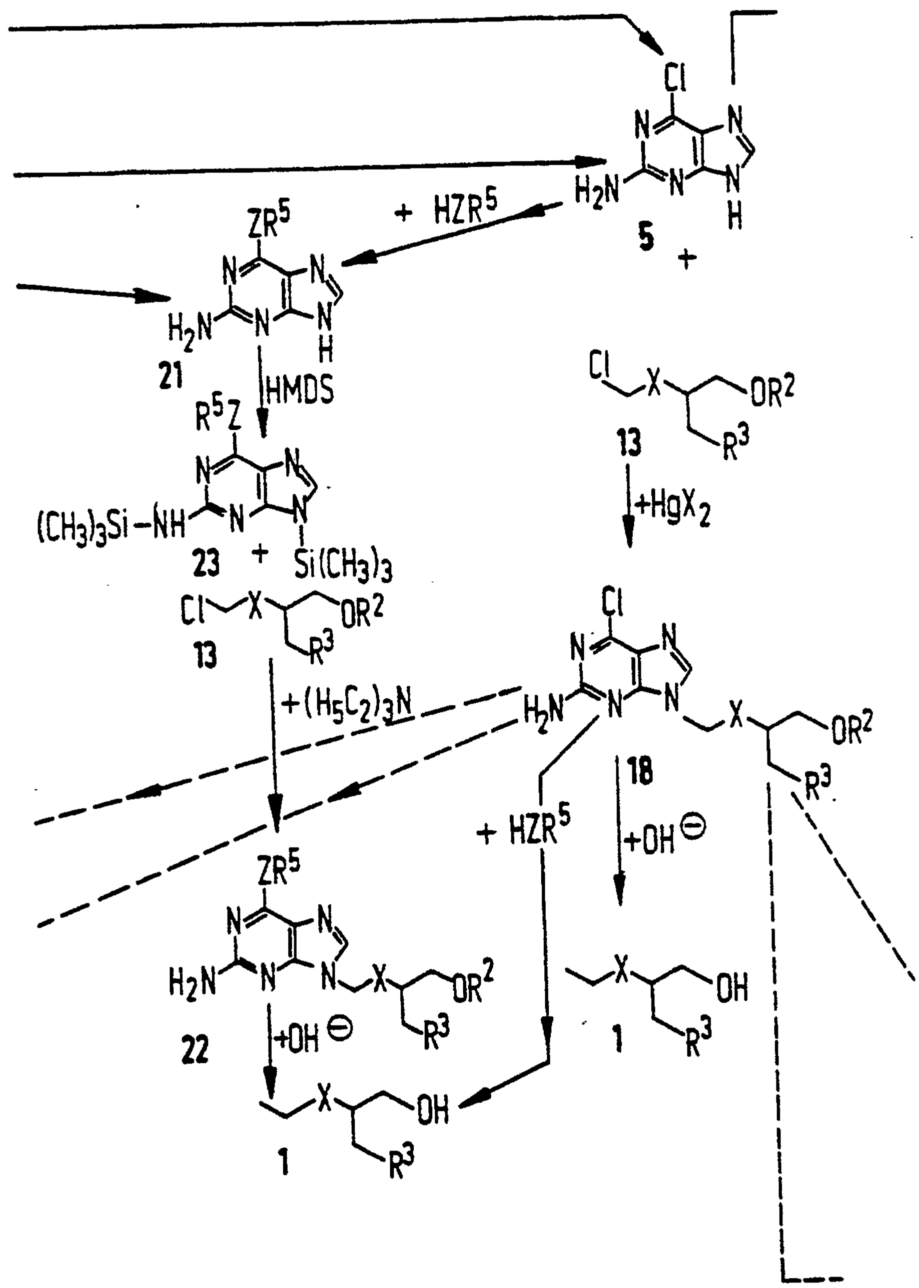


5 + 21 13 23 + 13 18 22 1 1

FIG. 2 / Teil 3

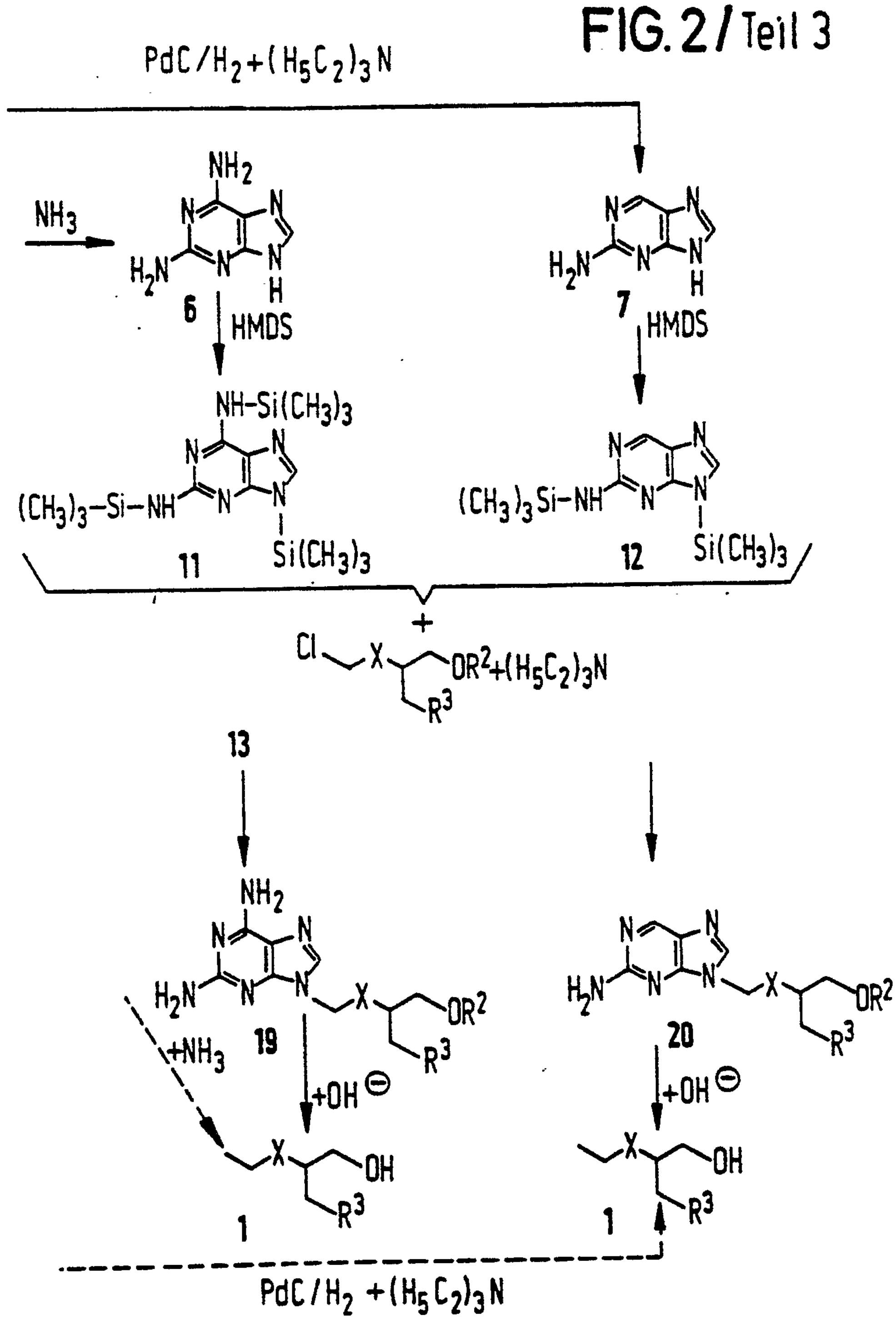


6, 7, 11, 12, 13, 19, 20, 1, 1

Europäisches Patentamt

# EUROPÄISCHER RECHERCHENBERICHT

Nummer der Anmeldung

EP 90113147.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| P,A | CHEMICAL ABSTRACTS, Band 110, Nr. 11, 13. März 1989, Columbus, Ohio, USA E. WINKELMANN et al. "New prodrugs of acyclic nucleosides with antiriral activity" Seite 18, Spalte 1, Zusammenfassung-Nr. 87 985m & Arzneim.-Forschung 1988, 38(11), 1545-8 -- | 1,3 | C 07 D 473/32<br>A 61 K 31/52 |
| A | EP - A2 - 0 085 424 (SYNTEX) * Ansprüche 1,11,16 * -- | 1-3 | |
| D,A | EP - A1 - 0 145 207 (WARNER-LAMBERT) * Ansprüche 1,12 * -- | 1,2 | |
| A | CH - A5 - 629 805 (WELLCOME) * Gesamt * -- | 1-3 | RECHERCHIERTE SACHGEBIETE (Int Cl 5) |
| D,A | EP - A2 - 0 152 965 (SYNTEX) * Anspruch 1 * -- | 1 | C 07 D 473/00 |
| D,A | JOURNAL OF MEDICINAL CHEMISTRY, Band 28, Nr. 3, März 1985 J.C. MARTIN et al. "Acyclic Analogues of 2'-Deoxynucleosides Related to 9-((1,3--Dihydroxy-2-propoxy)methyl) guanine as Potential Antiviral Agents" Seite 358-362 * Seite 358; Schemata III,IV * ---- | 1,2 | |

Der vorliegende Recherchenbericht wurde für alle Patentansprüche erstellt.

| Recherchenort | Abschlußdatum der Recherche | Prüfer |
|---|---|---|
| WIEN | 14-09-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-

EP 90113147.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
|---|---|---|---|
| | Refund: 75 % | | |

RECHERCHIERTE SACHGEBIETE (Int Cl.5)

Der vorliegende Recherchenbericht wurde für alle Patentansprüche erstellt.

| Recherchenort | Abschlußdatum der Recherche | Prüfer |
|---|---|---|
| WIEN | 14-09-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82